# FASCICULE DE BREVET EUROPEEN

(11) **EP 2 459 738 B1**
(45) Date de publication et mention de la délivrance du brevet: **29.10.2014**
(21) Numéro de dépôt: 10762956.0
(22) Date de dépôt: 29.07.2010
(51) Int. Cl.: C12Q 1/04, C12Q 1/26

(54) **NOUVEAUX SUBSTRATS ENZYMATIQUES DE NITROREDUCTASE**
NEUARTIGE INTROREDUKTASTE-ENZYMSUBSTRATE
NOVEL NITROREDUCTASE ENZYMATIC SUBSTRATES

(30) Priorité: 30.07.2009 FR 0903761
(43) Date de publication de la demande: 06.06.2012
(73) Titulaire: Biomérieux, 69280 Marcy L'etoile (FR)
(72) Inventeur: FABREGA, Olivier, Newcastle Upon Tyne NE3 3GJ (GB); JAMES, Arthur, Cumbria CA13 9 UX (GB); ORENGA, Sylvain, F-01160 Neuville Sur Ain (FR); PERRY, John, Newcastle upon Tyne NE7 7BH (GB); SALWATURA, Vindhya, Lakshika, Newcastle Upon Tyne NE4 9JB (GB); STANFORTH, Stephen, Northumberland NE 43 7EH (GB)
(74) Mandataire: Bitaud, Valérie Marie-Odile
(86) Numéro de dépôt international: PCT/FR2010/051623
(87) Numéro de publication internationale: WO 2011/012826

(56) Documents cités:
- WO-A1-2008/030120
- FR-A1- 2 785 620
- FR-A1- 2 916 762
- MANAFI M ET AL: "FLUOROGENIC AND CHROMOGENIC SUBSTRATES USED IN BACTERIAL DIAGNOSTICS", MICROBIOLOGICAL REVIEWS, AMERICAN SOCIETY FOR MICROBIOLOGY, WASHINGTON, DC, US, vol. 55, no. 3, 1 septembre 1991 (1991-09-01), pages 335-348, XP009020939, ISSN: 0146-0749
- MCCORMICK N G ET AL: "MICROBIAL TRANSFORMATION OF 2 4 6 TRI NITRO TOLUENE AND OTHER NITRO AROMATICCOMPOUNDS", APPLIED AND ENVIRONMENTAL MICROBIOLOGY, AMERICAN SOCIETY FOR MICROBIOLOGY, US, vol. 31, no. 6, 1 janvier 1976 (1976-01-01), pages 949-958, XP009095207, ISSN: 0099-2240

## Description

La présente invention concerne de nouveaux substrats enzymatiques pour la détection d'activité nitroréductase. Ces substrats sont utilisables dans les applications comportant une étape de réduction enzymatique produisant un signal physico-chimique notamment en microbiologie, biochimie, immunologie, biologie moléculaire, histologie, etc.

Il existe actuellement de très nombreux milieux permettant la détection de microorganismes. Cette détection peut être basée notamment sur l'utilisation de substrats particuliers, spécifiques d'une enzyme du microorganisme que l'on souhaite détecter. D'une manière générale, les substrats synthétiques d'enzymes sont constitués de telle façon que le substrat et le produit de son métabolisme par l'enzyme cible possèdent des propriétés physico-chimiques différentes, permettant de les distinguer et d'évaluer si tout ou partie du substrat a été converti en produit par l'enzyme. Par exemple le produit du métabolisme enzymatique peut être chromogène ou fluorescent. Ainsi dans le cas des bactéries, par le choix des substrats, selon qu'il y a réaction ou non, il est possible de caractériser la nature d'un microorganisme. Une activité nitroréductase peut notamment être utilisée pour révéler un groupe, un genre ou une espèce de bactéries. Il peut également être utilisé pour suivre le métabolisme réducteur de microorganismes, par exemple lié à leur croissance ou à l'inhibition de cette croissance.

La capacité de certaines bactéries de réduire les composés nitro-aromatiques est connue depuis de nombreuses années. Asnis (1957) a rapporté l'isolation d'une flavoproteine à partir d'extraits d'*E*. *coli* qui était capable de réduire l'acide p-nitrobenzoique. Depuis ce rapport, l'activité nitroaryl-réductase a été identifiée dans diverses variétés d'organismes. Ceci inclut des aérobies strictes tels que *Pseudomonas spp.* (Won et al. 1974) et *Nocardia spp.* (Villanueva 1964), des anaérobies strictes tels que *Clostridium spp.* (Ancermaier & Simon 1983) et *Veillonella spp.* (McCormick et al. 1976), ou encore des champignons (Masuda & Ozaki 1993) et des parasites eucaryotes (Douch 1975). II existe une gamme de substrats qui ont été désignés comme étant susceptibles d'être réduits par des nitroaryl-reductases bactériennes. Il s'agit surtout de composés nitro-aromatiques tels que l'acide p-nitrobenzoique, le p-nitrophénol, la p-nitroaniline et le 2,4,6-trinitrotoluène (McCormick et al. 1976).

D'une manière générale, la détection de l'activité enzymatique nitroréductase est réalisée par des méthodes indirectes telles que le suivi de la disparition du substrat ou d'un cofacteur. Par exemple, Kitamura et al. (1983) ont étudié la réduction du méthyl p-nitrobenzoate et d'une gamme d'autres composés aromatiques nitrés avec des extraits *d'E. coli.* Toutefois, cette méthode est peu sensible et n'est pas adaptée à une détection en milieu hétérogène. On peut citer également la demande WO 00/28073 qui décrit un substrat fluorogène à base de nitrocoumarine pour la détection directe des activités nitroaryl-réductases. Ce type de composé nitro-aromatique est capable de produire, après réduction, un composé très fluorescent qui est donc facilement détectable. Toutefois ce substrat n'est pas bien adapté à une détection en milieu hétérogène. D'autres substrats enzymatiques de nitroréductase sont décrits dans la demande de brevet FR2916762.
La présente invention se propose donc d'améliorer les substrats de nitroréductase permettant la détection de microorganismes. Comparativement aux substrats existants, ces nouveaux substrats sont de synthèse aisée, et peuvent être utilisés notamment en milieux gélifiés pour la détection de micro-organismes car ils produisent une coloration ne diffusant pas dans le milieu réactionnel.

En outre, la présente invention contribue à améliorer l'état de la technique en ce que les composés selon l'invention permettent la détection de microorganismes présentant conjointement une activité nitroréductase et un métabolisme entrainant une variation de pH du milieu.

Avant d'aller plus avant dans la description de l'invention, les définitions ci dessous sont données afin de faciliter l'exposé de l'invention.

Par substrat enzymatique, on entend un substrat pouvant être modifié par une enzyme en un produit permettant la détection, directe ou indirecte d'un microorganisme, d'une cellule ou d'une organelle. Dans le cas des substrats de nitroréductase, ce substrat comprend notamment une fonction nitrate qui est partiellement ou totalement réduite par l'activité enzymatique à révéler, la réduction de cette fonction nitrate modifiant certaines propriétés physicochimiques de la molécule, permettant de suivre cette réduction.

Les substrats selon l'invention sont adaptés à une utilisation en cytométrie en flux, car le produit de la réduction restant principalement localisé dans la cellule exprimant l'activité enzymatique, il est possible de compter spécifiquement les cellules exprimant cette activité, voire de les séparer du reste de l'échantillon.

Les substrats selon l'invention sont également bien adaptés à une utilisation en histoenzymologie, car le produit de la réduction restant principalement localisé sur le lieu de la réduction, il est possible d'identifier spécifiquement les cellules ou organelles exprimant cette activité au sein d'un tissu.

Du fait de leur faible toxicité, les substrats selon l'invention sont bien adaptés au suivi d'activité nitroréductase de culture cellulaire.

Les substrats selon l'invention sont particulièrement bien adaptés à une utilisation en milieu de détection et/ou d'identification car ils produisent une coloration ou une fluorescence ne diffusant pas dans le milieu réactionnel. Dans la présente demande, le terme coloration est employé pour couvrir une coloration dans le spectre visible, absorption de lumière, ou une fluorescence, absorption à une longueur d'onde (λₑₓ) et émission à une longueur d'onde supérieure (λₑₘ, λₑₘ > λₑₓ).

Les substrats de l'invention peuvent être salifiés, c'est à dire sous forme de sel tel que chlorure, bromure, iodure, potassium ou trifluoroacétate.

Par indicateur de pH, on entend une substance chimique dont la couleur et/ou la fluorescence varie en fonction des modifications de pH du milieu, lesdites modifications étant liées ou non au métabolisme du ou des microorganismes en croissance sur ledit milieu.

Par nitroréductase, on entend une enzyme pouvant réduire totalement ou partiellement un groupement NO₂.

Par groupement alkyle, on entend une chaîne de groupements hydrocarbonés saturés, tel que notamment un alkyle en C₁-C₆, c'est a dire un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.

Par groupement aryle, on entend un groupement fonctionnel (ou substituant) qui dérive d'un noyau aromatique tel que notamment un noyau aromatique en C₆-C₁₀, notamment phényle, benzyle, 1-naphtyle ou 2-naphtyle.

Par groupement carboxyle, on entend notamment un groupe fonctionnel composé d'un atome de carbone, lié par une double liaison à un premier atome d'oxygène, et par une liaison simple à un second atome d'oxygène, lui-même chargé négativement ou relié à un atome d'hydrogène. En fonction du pKₐ de la molécule et du pH du milieu, le groupement carboxyle peut être sous forme ionisée, c'est à dire sans H lié au second atome d'oxygène, qui est alors chargé négativement.

Par milieu réactionnel, on entend un milieu comprenant tous les éléments nécessaires à l'expression d'un métabolisme et/ou à la croissance de microorganismes, d'une cellule ou d'une organelle. Ce milieu réactionnel peut être utilisé en cytométrie en flux, histoenzymologie, culture cellulaire ... ou en tant que milieu de détection et/ou d'identification de microorganismes.

Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine ou de l'agarose. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

Le milieu réactionnel peut comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux.

Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant...

Le milieu réactionnel peut être un milieu de détection et/ou d'identification, c'est à dire un milieu de révélation, ou un milieu de culture et de révélation. Dans le premier cas, la culture des microorganismes est effectuée avant ensemencement et, dans le deuxième cas, le milieu de détection et/ou d'identification constitue également le milieu de culture.

Par échantillon biologique, on entend un échantillon clinique, issu d'un prélèvement de liquide biologique ou un échantillon alimentaire, issu de tout type d'aliment ou un échantillon cosmétique ou pharmaceutique issu de toute préparation cosmétique ou pharmaceutique. Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits; de yaourt, de viande, d'oeufs, de légumes, de mayonnaise, de fromage ; de poisson..., un échantillon alimentaire issu d'une alimentation destinée aux animaux, tel que notamment un échantillon issu de farines animales. L'échantillon peut également être issu d'un prélèvement de l'environnement clinique, d'élevage ou de production alimentaire, cosmétique ou pharmaceutique. Par prélèvement d'environnement, on entend notamment un prélèvement de surface, de liquide, de matière première ou de produit. Par échantillon, on entend donc le prélèvement en lui même (écouvillon, selles, aliments, ...) aussi bien que des colonies de microorganismes issus dudit prélèvement (par exemple après isolement sur un milieu de culture gélifié, ou dans un bouillon d'enrichissement ensemencé avec ledit prélèvement).

Au sens de la présente invention, le terme microorganisme recouvre les bactéries, notamment à Gram négatif et à Gram positif, les levures, les moisissures, et plus généralement, les organismes généralement unicellulaire, invisibles à l'oeil nu, qui peuvent être multipliés et manipulés en laboratoire.

A titre de bactéries à Gram négatif, on peut citer les bactéries des genres suivants : *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Citrobacter, Hafnia, Edwardsiella, Aeromonas, Moraxella; Pasteurella, Providencia, Actinobacillus, Alcaligenes, Bordetella, Cedecea, Erwinia, Pantoea, Ralstonia, Stenotrophomonas, Xanthomonas* et *Legionella.*

A titre de bactéries à Gram positif, on peut citer les bactéries des genres suivants : *Aerococcus, Enterococcus, Streptococcus, Staphylococcus, Bacillus, Lactobacillus, Listeria, Clostridium, Gardnerella, Kocuria, Lactococcus, Leuconostoc, Micrococcus, Falkamia, Gemella, Pediococcus, Mycobacterium* et *Corynebacterium.*

A titre de levures, on peut citer les levures des genres suivants : *Candida, Cryptococcus, Saccharomyces* et *Trichosporon.*

Préférentiellement, les microorganismes appartiennent aux genres *Escherichia, Shigella, Salmonella, Serratia, Enterobacter, Citrobacter, Klebsiella, Proteus, Providencia, Morganella, Yersinia, Vibrio, Pseudomonas, Acinetobacter, Enterococcus, Staphylococcus, Streptococcus, Listeria, Bacillus, Candida, Cryptococcus, Saccharomyces.*

A ce titre, l'invention concerne l'utilisation d'un composé de la formule (1) suivante, comme substrat enzymatique pour la détection d'une activité nitroréductase : 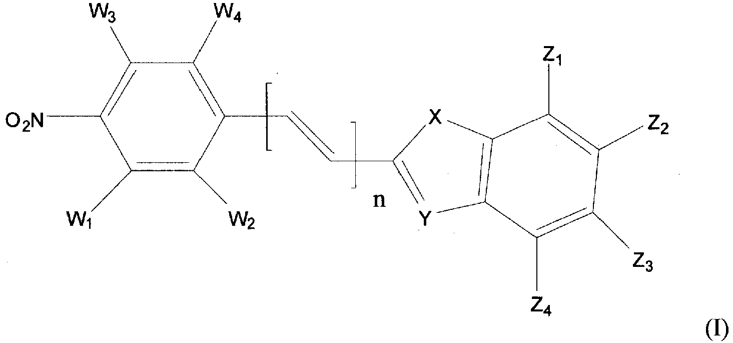 selon laquelle :
- W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
- n = 0, 1 ou 2
- X est NR, CZ₅Z₆, S ou O, R étant H, alkyle, aralkyle, aryle, alcanoïque ou alkylsulfonique, Z₅ et Z₆ étant un alkyle
- Y est N ou N⁺R, R étant alkyle, aralkyle, aryle, alcanoïque ou alkylsulfonique
- Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

Selon un autre mode de réalisation de l'invention, celle-ci concerne l'utilisation d'un composé de formule (I) telle qu'indiquée *supra,* comme substrat enzymatique pour la détection d'une activité nitroréductase, et indicateur d'une variation de pH.

Selon un mode préféré de réalisation de l'invention, n = 1.

Selon un mode préféré de réalisation de l'invention, W₁, W₂, W₃ et W₄ sont indépendamment H.

Selon un mode préféré de réalisation de l'invention, X est S ou CZ₅Z₆, préférentiellement CCH₃CH₃.

Selon un mode préféré de réalisation de l'invention, Y est N ou N⁺CH₃.

Selon un mode préféré de réalisation de l'invention, Z₁, Z₂, Z₃ et Z₄ sont H.

Selon un mode préféré de réalisation de l'invention, ledit composé est choisi parmi 2-(4'-Nitrostyryl)-benzothiazole, 2-(4'-Nitrostyryl)-N-methyl-benzothiazolium chloride, 2-(4'-Nitrostyryl)-1,3,3-triméthyl-indolinium dibromide.

L'invention concerne également un procédé pour la détection chez des micro-organismes d'une activité nitroréductase, caractérisé en ce qu'il comprend les étapes suivantes:
a) disposer d'un milieu de détection et/ou d'identification comprenant un composé de formule (I) 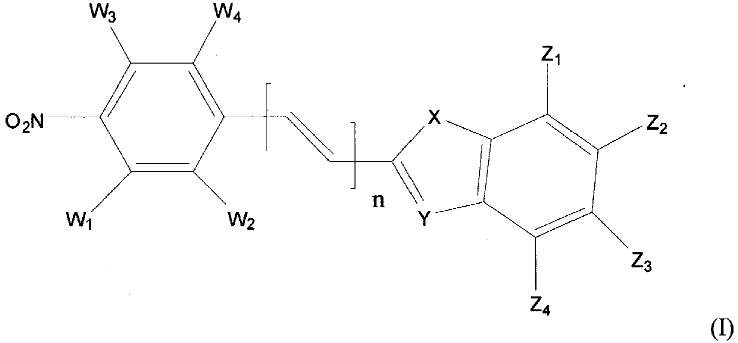 selon laquelle :
   - W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
   - n = 0, 1 ou 2
   - X est NR, CZ₅Z₆, S ou O, R étant H, alkyle, aralkyle, aryle, alcanoïque ou alkylsulfonique, Z₅ et Z₆ étant un alkyle
   - Y est N ou N⁺R, R étant alkyle, aralkyle, aryle, alcanoïque ou alkylsulfonique
   - Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
   et leurs sels ;
b) ensemencer le milieu avec un échantillon biologique à tester,
c) laisser incuber, et
d) révéler la présence d'au moins une activité nitroréductase.

Selon un autre mode de réalisation de l'invention, celle-ci concerne également un procédé pour la détection d'une activité nitroréductase chez des microorganismes, et d'une variation de pH, caractérisé en ce qu'il comprend les étapes suivantes :
a) disposer d'un milieu de détection et/ou d'identification comprenant un composé de formule (I) telle qu'indiquée *supra ;*
b) ensemencer le milieu avec un échantillon biologique à tester ;
c) laisser incuber ;
d) observer un changement de couleur et/ou de fluorescence du milieu de détection et/ou d'identification, révélant la présence d'au moins une activité nitroréductase, et une variation de pH dans ledit milieu de détection et/ou d'identification.

Selon un mode préféré de réalisation de l'invention, n = 1.

Selon un mode préféré de réalisation de l'invention, W₁, W₂, W₃ et W₄ sont indépendamment H.

Selon un mode préféré de réalisation de l'invention, X est S ou CZ₅Z₆, préférentiellement CCH₃CH₃.

Selon un mode préféré de réalisation de l'invention, Y est N ou N⁺CH₃.

Selon un mode préféré de réalisation de l'invention, Z₁, Z₂, Z₃ et Z₄ sont H.

Selon un mode préféré de réalisation de l'invention, ledit composé est choisi parmi 2-(4'-Nitrostyryl)-benzothiazole, 2-(4'-Nitrostyryl)-N-methyl-benzothiazolium chloride, 2-(4'-Nitrostyryl)-1,3,3-triméthyl-indolinium dibromide.

L'ensemencement des microorganismes peut être réalisé par toutes les techniques d'ensemencement connues de l'homme du métier. Une étape d'incubation peut être réalisée à une température pour laquelle l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité nitroréductase, seule ou en combinaison avec au moins une autre activité enzymatique.

L'invention concerne également un milieu de détection et/ou d'identification de microorganismes comprenant un composé de la formule (I) suivante: 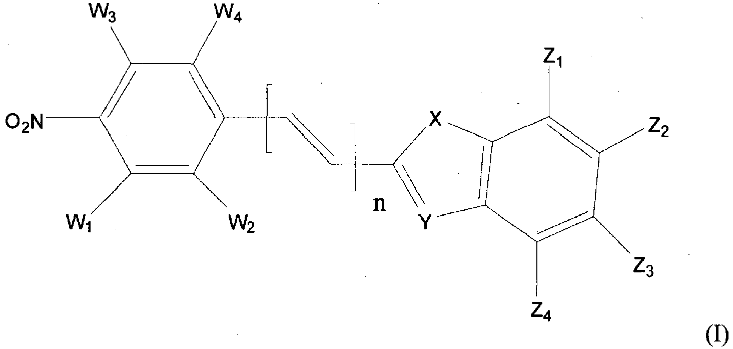 selon laquelle :
- W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
- n = 0, 1 ou 2
- X est NR, CZ₅Z₆, S ou O, R étant H, alkyle, aralkyle, aryle, alcanoïque ou alkylsulfonique, Z₅ et Z₆ étant un alkyle
- Y est N ou N⁺R, R étant alkyle, aralkyle, aryle, alcanoïque ou alkylsulfonique
- Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

Selon un mode préféré de réalisation de l'invention, n = 1.

Selon un mode préféré de réalisation de l'invention, W₁, W₂, W₃ et W₄ sont indépendamment H.

Selon un mode préféré de réalisation de l'invention, X est S ou CZ₅Z₆, préférentiellement CCH₃CH₃.

Selon un mode préféré de réalisation de l'invention, Y est N ou N⁺CH₃.

Selon un mode préféré de réalisation de l'invention, Z₁, Z₂, Z₃ et Z₄ sont H.

Selon un mode préféré de réalisation de l'invention, ledit composé est choisi parmi 2-(4'-Nitrostyryl)-benzothiazole, 2-(4'-Nitrostyryl)-N-methyl-benzothiazolium chloride, 2-(4'-Nitrostyryl)-1,3,3-triméthyl-indolinium dibromide.

Préférentiellement, ledit milieu réactionnel est un milieu de détection et/ou d'identification de microorganismes, ledit milieu comprenant au moins une molécule utilisée à titre de substrat enzymatique telle que définie ci avant.

Préférentiellement, ledit composé est à une concentration comprise entre 1 et 1000 mg/l, préférentiellement entre 10 et 500 mg/l.

Préférentiellement, ledit milieu réactionnel est un milieu de détection et/ou d'identification de microorganismes, ledit milieu comprenant en outre au moins un composé dont le métabolisme entraîne une variation de pH. Préférentiellement ledit composé est un sucre, un acide aminé ou un acide organique. Préférentiellement ledit composé est un pentose, un hexose, un dioside, un trioside ou un polyoside. Préférentiellement, ledit composé est à une concentration comprise entre 0,1 et 100 g/l, préférentiellement entre 1 et 50 g/l. Préférentiellement, ledit composé est à une concentration comprise entre 5 et 30 g/l.

Selon un mode particulier de réalisation de l'invention, ledit milieu de détection et/ou d'identification selon l'invention comprend en outre au moins un autre substrat enzymatique, spécifique d'une activité enzymatique différente de l'activité nitroréductase détectée par la molécule selon l'invention.

Le métabolisme enzymatique du ou des autres substrats génère un signal détectable, différent du signal généré par le composé selon l'invention utilisé à titre de substrat enzymatique, comme par exemple des produits colorés ou fluorescents différents, pour permettre la mise en évidence comme la détection et/ou l'identification et/ou la quantification d'un ou plusieurs microorganismes. A titre d'autre substrat spécifique, on peut utiliser tout autre substrat classiquement utilisé dans la détection des microorganismes. La concentration de l'autre substrat enzymatique spécifique est généralement comprise entre 0,01 et 1 g/l. L'homme du métier pourra déterminer facilement une telle concentration en fonction du substrat utilisé. A titre indicatif, il est possible de combiner les composés selon l'invention avec des substrats enzymatiques de peptidase, d'osidase, d'estérase ou de réductase.

Selon un mode particulier de réalisation de l'invention, ledit milieu de détection et/ou d'identification selon l'invention comprend en outre au moins un autre substrat enzymatique spécifique de l'activité nitroréducase. Par le choix particulier de substrats, il est alors possible d'identifier des groupes de microorganismes exprimant une même activité enzymatique. La concentration de l'autre substrat enzymatique spécifique est généralement comprise entre 0,01 et 1 g/l. L'homme du métier pourra déterminer facilement une telle concentration en fonction du substrat utilisé.

Selon un mode particulier de réalisation de l'invention, ledit milieu de détection et/ou d'identification selon l'invention comprend en outre au moins un indicateur métabolique, spécifique d'une activité métabolique différente de celle détectée par le composé selon l'invention.

Cet indicateur métabolique peut notamment être une source de Carbone ou d'Azote associée ou non à un réactif révélant son métabolisme.

Les exemples ci dessous sont donnés à titre explicatif et n'ont aucun caractère limitatif. Ils permettront de mieux comprendre l'invention.

### EXEMPLES

### Exemple - Utilisation de substrats de formule I selon l'invention pour détecter une activité nitroréductase

### a) Substrats de nitroréductase

Le 2-(4-Nitrostyryl)-1,3,3-trimethyl-indolinium iodé, a été synthétisé à partir de 1,3,3-Triméthyle-indolinium iodé suivant la méthode de Bahner *et al.* (C. Bahner, J. Dale, J. Fain, E. Franklin, J. Goan, W. Stump, M. West et J. Wilson, Journal of Organic Chemistry, 1957, 22, 1110).

### b) Préparation du milieu

40 mg du substrat ont été dissous dans 4ml de Diméthylsulfoxide et ajoutés à 400 ml de milieu Columbia préalablement autoclavé. Le milieu a été réparti en boîte de Pétri de 90 mm de diamètre à raison de 20 ml par boîte.

### c) Ensemencement et incubation

Dix souches de micro-organismes issues de collections et appartenant à différentes espèces de bactéries et levures sont ensemencées en spot d'environ 10 000 unités formant colonies.

Les milieux sont incubés 24 heures à 37°C, puis les colonies formées sont examinées visuellement.

### d) Lecture des résultats

Les résultats obtenus sont présentés dans le tableau suivant.

| | Croissance | Coloration |
|---|---|---|
| E. coli NCTC 10418 | ++ | - |
| S. marcescens NCTC 10211 | ++ | - |
| Ps. aeruginosa NCTC 10662 | ++ | - |
| B. cepacia 1222 | + | - |
| Y. enterocolitica NCTC 11176 | ++ | - |
| S. typhimurium NCTC 74 | + | - |
| C. freundii 46262 (wild) | ++ | - |
| M. morganii 462403 (wild) | ++ | - |
| E. cloacae NCTC 11936 | ++ | Rose pâle |
| P. rettgeri NCTC 7475 | ++ | Rose pâle |

### e) Conclusions

Sur les milieux selon l'invention, il est possible de détecter une activité nitroréductase de micro-organismes *E*. *cloacae* et *P. rettgeri* grâce à la coloration des colonies.

Par les différentes variations de structure des substrats de nitroréductase selon l'invention, il est possible de distinguer différents groupes de microorganismes et d'obtenir différentes couleurs de colonies. De plus, la coloration ne diffusant pas dans le milieu réactionnel, il est possible de distinguer et de compter les cellules ou colonies exprimant l'activité nitroréductase indépendamment de celles ne l'exprimant pas

## Revendications

1. Utilisation d'un composé de la formule (I) suivante, comme substrat enzymatique pour la détection d'une activité nitroréductase : 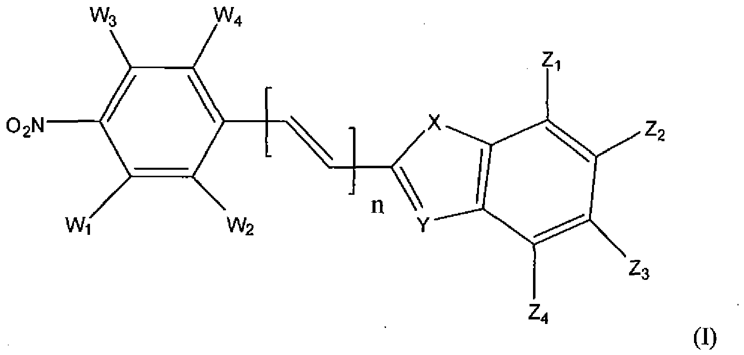 selon laquelle :
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
• n = 0, 1 ou 2
• X est NR, CZ₅Z₆, S ou O, R étant H, alkyle, aralkyle, aryle, alcanoïque ou alkylsulfonique, Z₅ et Z₆ étant un alkyle
• Y est N ou N⁺R, R étant alkyle, aralkyle, aryle, alcanoïque ou alkylsulfonique
• Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

2. Utilisation d'un composé de formule (I) suivante, comme substrat enzymatique pour la détection d'une activité nitroréductase et indicateur d'une variation de pH : 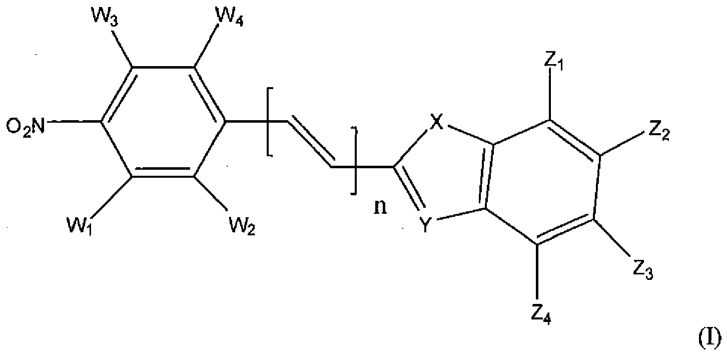 selon laquelle :
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
• n = 0, 1 ou 2
• X est NR, CZ₅Z₆, S ou O, R étant H, alkyle, aralkyle, aryle, alcanoïque ou alkylsulfonique, Z₅ et Z₆ étant un alkyle
• Y est N ou N⁺R, R étant alkyle, aralkyle, aryle, alcanoïque ou alkylsulfonique a Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

3. Utilisation d'un composé de formule (I) selon la revendication 1 ou 2 selon laquelle n = 1.

4. Utilisation d'un composé de formule (I) selon l'une des revendications précédentes selon laquelle W₁, W₂, W₃ et W₄ sont indépendamment H

5. Utilisation d'un composé de formule (I) selon l'une quelconque des revendications précédentes selon laquelle X est S ou CZ₅Z₆, préférentiellement CCH₃CH₃.

6. Utilisation d'un composé de formule (I) selon l'une quelconque des revendications précédentes selon laquelle Y est N ou N⁺CH₃.

7. Utilisation d'un composé de formule (I) selon l'une quelconque des revendications précédentes selon laquelle Z₁, Z₂, Z₃ et Z₄ sont H.

8. Utilisation d'un composé de formule (I) selon la revendication 1 ou 2 selon laquelle ledit composé est choisi parmi 2-(4'-Nitrostyryl)-benzothiazole, 2-(4'-Nitrostyryl)-N-methyl-benzothiazolium chloride, 2-(4'-Nitrostyryl)-1,3,3-triméthyl-indolinium dibromide.

9. Procédé pour la détection chez des micro-organismes d'une activité nitroréductase, **caractérisé en ce qu'**il comprend les étapes suivantes:
a) disposer d'un milieu de détection et/ou d'identification comprenant un composé de formule (I) 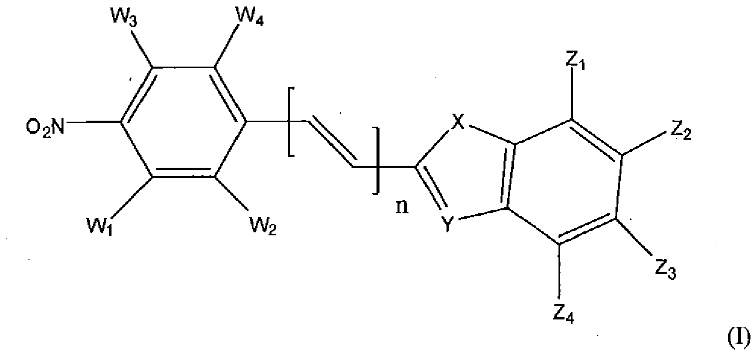 selon laquelle :
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
• n = 0, 1 ou 2
• X est NR, CZ₅Z₆, S ou O, R étant H, alkyle, aralkyle, aryle, alcanoïque ou alkylsulfonique, Z₅ et Z₆ étant un alkyle
• Y est N ou N⁺R, R étant alkyle, aralkyle, aryle, akanoïque ou alkylsulfonique
• Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels ;
b) ensemencer le milieu avec un échantillon biologique à tester,
c) laisser incuber, et
d) révéler la présence d'au moins une activité nitroréductase.

10. Procédé pour la détection d'une activité nitroréductase chez des microorganismes et d'une variation de pH, **caractérisé en ce qu'**il comprend les étapes suivantes :
a) disposer d'un milieu de détection et/ou d'identification comprenant un composé de formule (I) 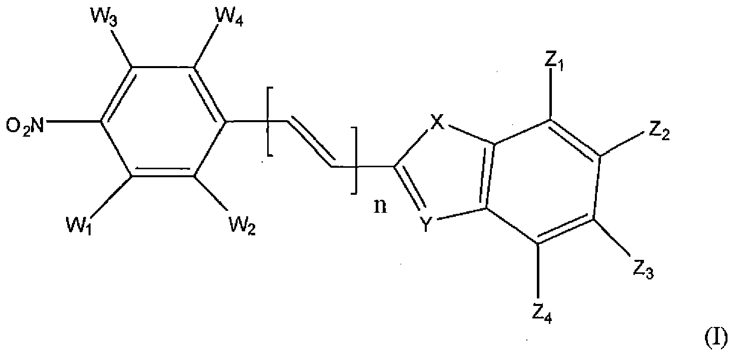 selon laquelle :
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
• n = 0, 1 ou 2
• X est NR, CZ₅Z₆, S ou O, R étant H, alkyle, aralkyle, aryle, alcanoïque ou alkylsulfonique, Z₅ et Z₆ étant un alkyle
• Y est N ou N⁺R, R étant alkyle, aralkyle, aryle, alcanoïque ou alkylsulfonique
• Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels ;
b) ensemencer le milieu avec un échantillon biologique à tester,
c) laisser incuber, et
d) observer un changement de couleur et/ou de fluorescence du milieu de détection et/ou d'identification, révélant la présence d'au moins une activité nitroréductase, et une variation de pH dans ledit milieu.

11. Milieu de détection et/ou d'identification de microorganismes comprenant un composé de la formule (I) suivante: 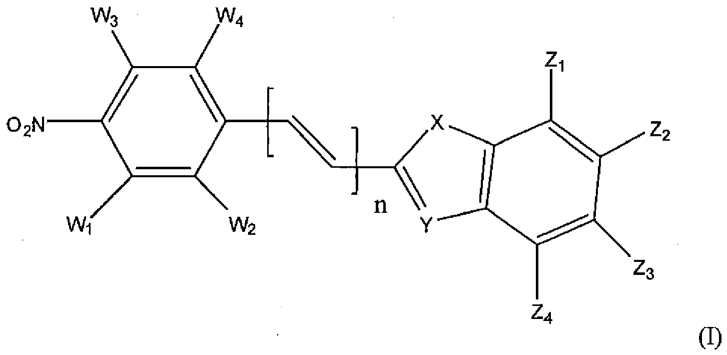 selon laquelle :
• W₁, W₂, W₃ et W₄, sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
• n = 0, 1 ou 2
• X est NR, CZ₅Z₆, S ou O, R étant H, alkyle, aralkyle, aryle, alcanoïque ou alkylsulfonique, Z₅ et Z₆ étant un alkyle
• Y est N ou N⁺R, R étant alkyle, aralkyle, aryle, alcanoïque ou alkylsulfonique
• Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

12. Milieu de détection et/ou d'identification de microorganismes selon la revendication 11, comprenant en outre au moins un composé dont le métabolisme entraîne une variation de pH dudit milieu, ledit composé étant un sucre, un acide aminé ou un acide organique.

## Patentansprüche

1. Verwendung einer Verbindung der folgenden Formel (I) als Enzymsubstrat zum Nachweis einer Nitroreduktase-Aktivität: 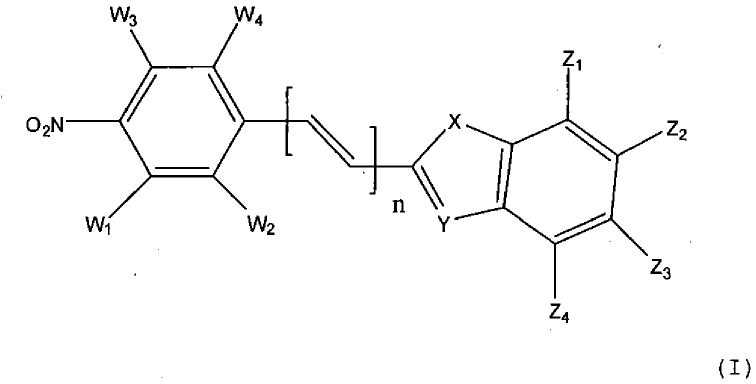 worin:
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• X für NR, CZ₅Z₆, S oder O steht, R für H, Alkyl, Aralkyl, Aryl, Alkanoyl oder Alkylsulfonyl steht, Z₅ und Z₆ für ein Alkyl stehen
• Y für N oder N⁺R steht, R steht für Alkyl, Aralkyl, Aryl, Alkanoyl oder Alkylsulfonyl
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze.

2. Verwendung einer Verbindung der Formel I als Enzymsubstrat zum Nachweis einer Nitroreduktase-Aktivität und Anzeiger einer Veränderung des pH-Werts : 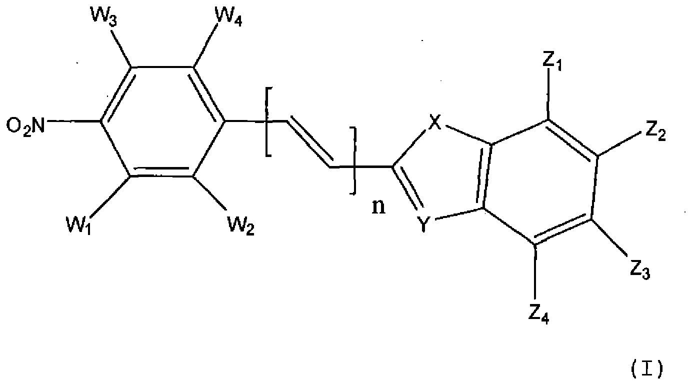 worin:
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• X für NR, CZ₅Z₆, S oder 0 steht, R für H, Alkyl, Aralkyl, Aryl, Alkanoyl oder Alkylsulfonyl steht, Z₅ und Z₆ für ein Alkyl stehen
• Y für N oder N⁺R steht,R steht für Alkyl, Aralkyl, Aryl, Alkanoyl oder Alkylsulfonyl
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze.

3. Verwendung einer Verbindung der Formel I nach Anspruch 1 oder 2, wobei n = 1.

4. Verwendung einer Verbindung der Formel I nach einem der vorigen Ansprüche, wobei W₁, W₂, W₃ und W₄ unabhängig für H stehen.

5. Verwendung einer Verbindung der Formel I nach einem der vorigen Ansprüche, wobei X für S oder CZ₅Z₆, vorzugsweise CCH₃CH₃, steht,

6. Verwendung einer Verbindung der Formel I nach einem der vorigen Ansprüche, wobei Y für N oder N⁺CH₃, steht.

7. Verwendung einer Verbindung der Formel I nach einem der vorigen Ansprüche, wobei Z₁, Z₂, Z₃ und Z₄ für H stehen.

8. Verwendung einer Verbindung der Formel I nach Anspruch 1 oder 2, wobei diese Verbindung aus Folgenden ausgewählt wird: 2-(4'nitrostyryl)benzothiazole,2- (4'nitrostyryl)-N-methylbenzothiazolium chloride,2-(4'nitrostyryl-1,3,3-trimethylindolinium dibromide.

9. Verfahren zum Nachweis einer Nitroreduktase-Aktivität in Mikroorganismen, dass die folgenden Schritte umfasst:
a) Bereitstellen eines Nachweis- und/oder Identifikationsmediums, umfassend eine Verbindung der folgenden Formel (I): 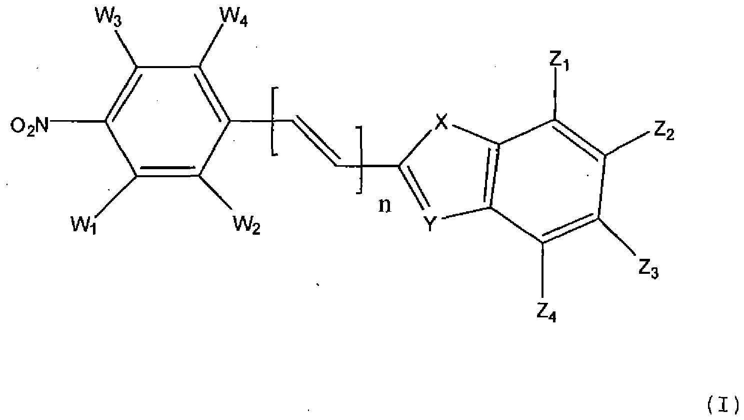 worin:
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• X für NR, CZ₅Z₆, S oder 0 steht, R für H, Alkyl, Aralkyl, Aryl, Alkanoyl oder Alkylsulfonyl steht, Z₅ und Z₆ für ein Alkyl stehen
• Y für N oder N⁺R steht,R steht für Alkyl, Aralkyl, Aryl, Alkanoyl oder Alkylsulfonyl
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze;
b) Beimpfen des Mediums mit einer zu testenden biologischen Probe,
c) Inkubieren und
d) Bestimmen der Anwesenheit von mindestens einer Nitroreduktase-Aktivität.

10. Medium zum Nachweis einer Nitroreduktase-Aktivität in Mikroorganismen und einer Veränderung des pH-Werts, dass die folgenden Schritte umfasst :
a) Bereitstellen eines Nachweis- und/oder Identifikationsmediums, umfassend eine Verbindung der folgenden Formel (I): 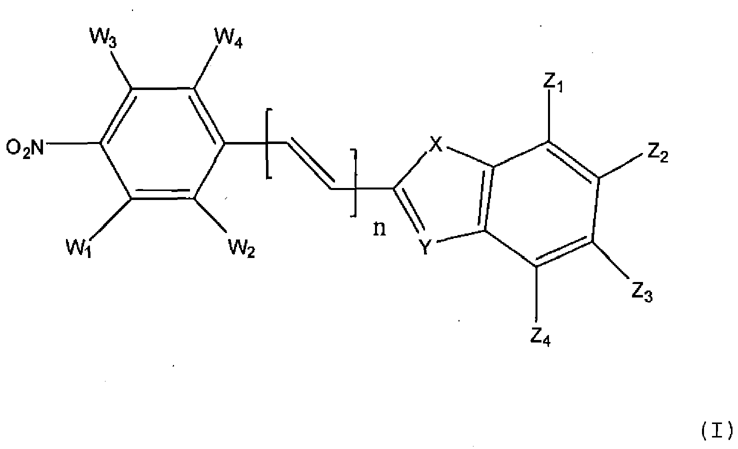 worin:
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• X für NR, CZ₅Z₆, S oder 0 steht, R für H, Alkyl, Aralkyl, Aryl, Alkanoyl oder Alkylsulfonyl steht, Z₅ und Z₆ für ein Alkyl stehen
• Y für N oder N⁺R steht,R steht für Alkyl, Aralkyl, Aryl, Alkanoyl oder Alkylsulfonyl
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze;
b) Beimpfen des Mediums mit einer zu testenden biologischen Probe,
c) Inkubieren und
d) Beobachtung einer Modifikation in der Farbe oder in der Fluoreszenz der Detektions- und/oder Identifikationsmedium, die zeigt die Anwesenheit von mindestens einer Nitroreduktase-Aktivität und einer pH Veränderung im Medium.

11. Medium zum Nachweis und/oder Identifikation von Mikroorganismen, umfassend eine Verbindung der folgenden Formel (I): 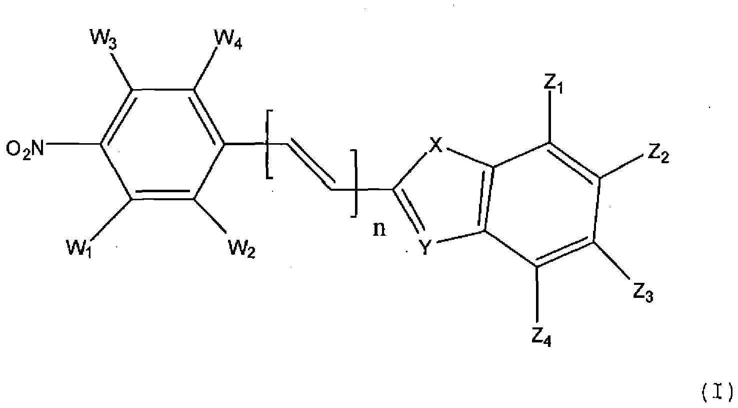 worin:
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• X für NR, CZ₅Z₆, S oder 0 steht, R für H, Alkyl, Aralkyl, Aryl, Alkanoyl oder Alkylsulfonyl steht, Z₅ und Z₆ für ein Alkyl stehen
• Y für N oder N⁺R steht,R steht für Alkyl, Aralkyl, Aryl, Alkanoyl oder Alkylsulfonyl
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze.

12. Das Medium zum Nachweis und/oder Identifikation von Mikroorganismen, nach Anspruch 11, zusätzlich umfassend mindestens eine Verbindung derem Metabolismus eine pH Veränderung dieses Mediums verursacht, diese Verbindung für eine Zucker, ein Amino-Säure oder ein Organische Säure steht.

## Claims

1. A use of a compound of the following formula (I) as enzyme substrate for the detection of a nitroreductase activity: 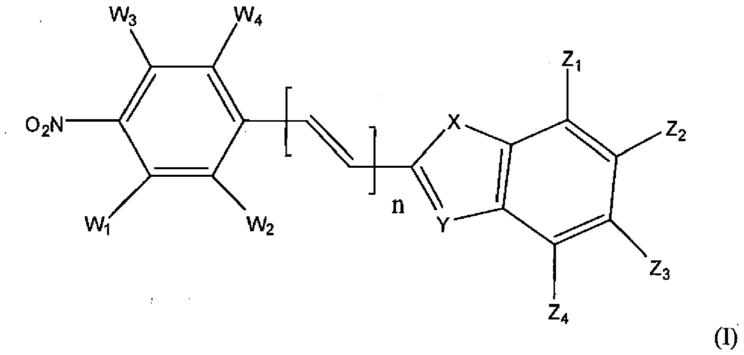 wherein:
• W₁, W₂, W₃ and W₄ are independently H, Br, Cl, F, I, alkyl, alkoxy, thiomethyl, perfluoroalkyl, nitro, cyano, carboxyl (including its esters or amides) or any combination thereof,
• n = 0, 1 or 2
• X is NR, CZ₅Z₆, S or O, R being H, alkyl, aralkyl, aryl, alkanoic or alkylsulfonic, Z₅ and Z₆ being an alkyl
• Y is N or N⁺R, R being alkyl, aralkyl, aryl, alkanoic or alkylsulfonic
• Z₁, Z₂, Z₃ and Z₄ being independently H, Br, Cl, F, I, alkyl, aryl, alkoxy, perfluoroalkyl, nitro, cyano, carboxyl, sulfonyl, including the esters or amides of carboxyl or sulfonyl
and salts thereof.

2. A use of a compound of the following formula (I) as enzyme substrate for the detection of a nitroreductase activity and indicator of a pH variation: 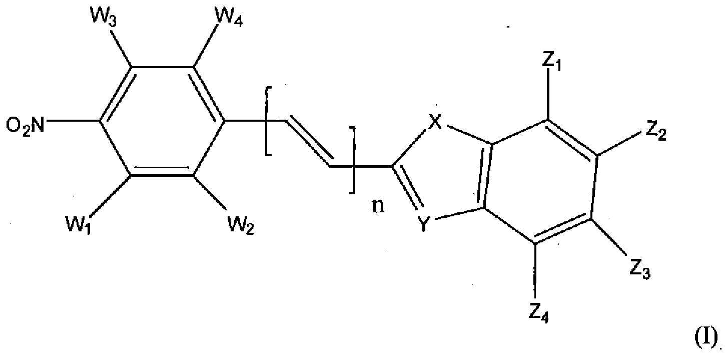 wherein:
• W₁, W₂, W₃ and W₄ are independently H, Br, Cl, F, I, alkyl, alkoxy, thiomethyl, perfluoroalkyl, nitro, cyano, carboxyl (including its esters or amides) or any combination thereof;
• n = 0, 1 or 2
• X is NR, CZ₅Z₆, S or O, R being H, alkyl, aralkyl, aryl, alkanoic or alkylsulfonic, Z₅ and Z₆ being an alkyl
• Y is N or N⁺R, R being alkyl, aralkyl, aryl, alkanoic or alkylsulfonic
• Z₁, Z₂, Z₃ and Z₄ being independently H, Br, Cl, F, I, alkyl, aryl, alkoxy, perfluoroalkyl, nitro, cyano, carboxyl, sulfonyl, including the esters or amides of carboxyl or sulfonyl
and salts thereof.

3. The use of a compound of formula (I) as claimed in claim 1 or 2, wherein n = 1.

4. The use of a compound of formula (I) as claimed in any one of the preceding claims, wherein W₁, W₂, W₃ and W₄ are independently H.

5. The use of a compound of formula (I) as claimed in any one of the preceding claims, wherein X is S or CZ₅Z₆, preferably CCH₃CH₃.

6. The use of a compound of formula (I) as claimed in any one of the preceding claims, wherein Y is N or N⁺CH₃.

7. The use of a compound of formula (I) as claimed in any one of the preceding claims, wherein Z₁, Z₂, Z₃ and Z₄ are H.

8. The use of a compound of formula (I) as claimed in claim 1 or 2, wherein said compound is chosen from 2-(4'-nitrostyryl)benzothiazole, 2-(4'-nitrostyryl)-N-methylbenzothiazolium chloride, 2-(4'-nitrostyryl-1,3,3-trimethylindoiinium dibromide.

9. A method of detecting in microorganisms a nitroreductase activity, comprising the following steps:
a) providing a detection and/or identification medium comprising a compound of formula (I) 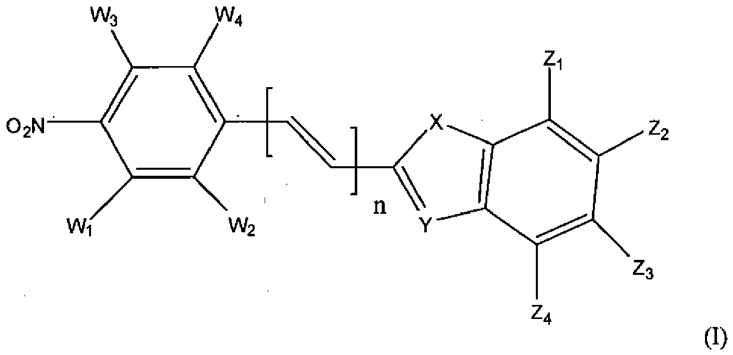 wherein:
• W₁, W₂, W₃ and W4 are independently H, Br, Cl, F, I, alkyl, alkoxy, thiomethyl, perfluoroalkyl, nitro, cyano, carboxyl (including its esters or amides) or any combination thereof
• n = 0, 1 or 2
• X is NR, CZ₅Z₆, S or O, R being H, alkyl, aralkyl, aryl, alkanoic or alkylsulfonic, Z₅ and Z₆ being an alkyl
• Y is N or N⁺R, R being alkyl, aralkyl, aryl, alkanoic or alkylsulfonic
• Z₁, Z₂, Z₃ and Z₄ being independently H, Br, Cl, F, I, alkyl, aryl, alkoxy, perfluoroalkyl, nitro, cyano, carboxyl, sulfonyl, including the esters or amides of carboxyl or sulfonyl
and salts thereof;
b) inoculating the medium with a biological sample to be tested,
c) leaving to incubate, and
d) detecting the presence of at least one nitroreductase activity.

10. A method of detecting in microorganisms a nitroreductase activity and a pH variation, comprising the following steps:
a) providing a detection and/or identification medium comprising a compound of formula (I) 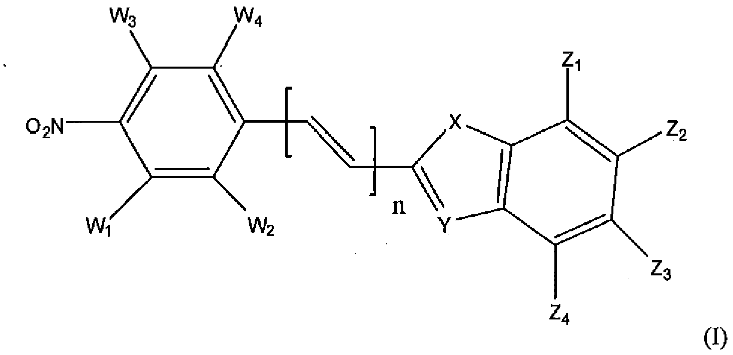 wherein:
• W₁, W₂, W₃ and W₄ are independently H, Br, Cl, F, I, alkyl, alkoxy, thiomethyl, perfluoroalkyl, nitro, cyano, carboxyl (including its esters or amides) or any combination thereof
• n = 0, 1 or 2
• X is NR, CZ₅Z₆, S or O, R being H, alkyl, aralkyl, aryl, alkanoic or alkylsulfonic, Z₅ and Z₆ being an alkyl
• Y is N or N⁺R, R being alkyl, aralkyl, aryl, alkanoic or alkylsulfonic
• Z₁, Z₂, Z₃ and Z₄ being independently H, Br, Cl, F, I, alkyl, aryl, alkoxy, perfluoroalkyl, nitro, cyano, carboxyl, sulfonyl, including the esters or amides of carboxyl or sulfonyl
and salts thereof;
b) inoculating the medium with a biological sample to be tested,
c) leaving to incubate, and
d) observing a change in color or in the fluorescence of the detection and/or identification medium, which reveals the presence of at least one nitroreductase activity and a pH variation in the medium.

11. A medium for the detection and/or identification of microorganisms comprising a compound of the following formula (I): 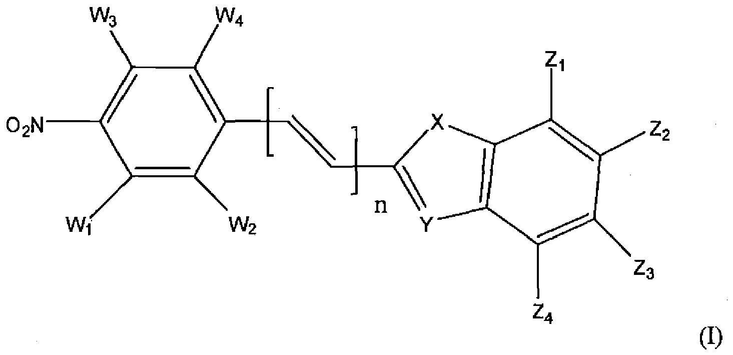 wherein:
• W₁, W₂, W₃ and W₄ are independently H, Br, Cl, F, I, alkyl, alkoxy, thiomethyl, perfluoroalkyl, nitro, cyano, carboxyl (including its esters or amides) or any combination thereof,
• n = 0, 1 or 2
• X is NR, CZ₅Z₆, S or O, R being H, alkyl, aralkyl, aryl, alkanoic or alkylsulfonic, Z₅ and Z₆ being an alkyl
• Y is N or N⁺R, R being alkyl, aralkyl, aryl alkanoic or alkylsulfonic
• Z₁, Z₂, Z₃ and Z₄ being independently H, Br, Cl, F, I, alkyl, aryl, alkoxy, perfluoroalkyl, nitro, cyano, carboxyl, sulfonyl, including the esters or amides of carboxyl or sulfonyl
and salts thereof.

12. The medium for the detection and/or identification of microorganisms as claimed in claim 11, additionally comprising at least one compound whose metabolism causes a pH variation of said medium, said compound being a sugar, an amino acid or an organic acid.
